# EUROPÄISCHE PATENTSCHRIFT

(11) **EP 1 421 076 B1**
(45) Veröffentlichungstag und Bekanntmachung des Hinweises auf die Patenterteilung: **10.10.2007**
(21) Anmeldenummer: 02758447.3
(22) Anmeldetag: 08.08.2002
(51) Int. Cl.: C07D 401/12

(54) **HYDRATE VON GEGEBENENFALLS SUBSTITUIERTEN 2-(2-PYRIDINYL)METHYLTHIO-1H-BENZIMIDAZOLEN UND VERFAHREN ZU IHRER HERSTELLUNG**
HYDRATES OF OPTIONALLY SUBSTITUTED 2-(2-PYRIDINYL)METHYLTHIO-1H-BENZIMIDAZOLES AND METHOD FOR THE PRODUCTION THEREOF
HYDRATES DE 2-(2-PYRIDINYL)METHYLTHIO-1H-BENZIMIDAZOLES EVENTUELLEMENT SUBSTITUES ET PROCEDES DE PRODUCTION DE CEUX-CI

(30) Priorität: 17.08.2001 DE 10140492
(43) Veröffentlichungstag der Anmeldung: 26.05.2004
(73) Patentinhaber: Grünenthal GmbH, 52078 Aachen (DE)
(72) Erfinder: LÖBERMANN, Hartmut, 52078 Aachen (DE); CASTER, Karl-Heinz, 52249 Eschweiler (DE)
(74) Vertreter: Kutzenberger, Helga
(86) Internationale Anmeldenummer: PCT/EP2002/008867
(87) Internationale Veröffentlichungsnummer: WO 2003/016301

(56) Entgegenhaltungen:
- EP-A- 0 074 241
- EP-A- 0 899 268
- KOTAR-JORDAN, B. ET AL: "Solid state characterization of K-1252" FARMACEVTSKI VESTNIK (LJUBLJANA) (1997), 48(POS. STEV.), 288-289 , XP001120145

## Beschreibung

Die Erfindung betrifft Kristalle aus gegebenenfalls substituierten 2-(2-Pyridinyl)methylthio-1 H-benzimidazol-Hydraten und Verfahren zu ihrer Herstellung.

Es ist bekannt, daß 2-(2-Pyridinyl)methylthio-1 H-benzimidazolverbindungen, wie z.B. Pyrmetazol (5-Methoxy-2-[(4-methoxy-3,5-dimethyl-2-pyridinyl)methylthio]-1 H-benzimidazol) die letzte Ausgangsstufe zur Herstellung von Ulkustherapeutika, insbesondere Omeprazol oder Lanzoprazol, sind. Zur Herstellung eines solchen Ulkustherapeutikums wird die Sulfidverbindung, wie z.B. Pyrmetazol, durch Oxidation in die entsprechende Sulfinylverbindung, wie z.B. Omeprazol, überführt.

Substituierte 2-(2-Pyridinyl)methylthio-1 H-benzimidazole werden üblicherweise durch Reaktion von Mercaptobenzimidazolverbindungen, wie z.B. 5-Methoxy-2-mercaptobenzimidazol, mit reaktiven Pyridinverbindungen, wie z.B. 2-Chlormethyl-3,5-dimethyl-4-methoxypyridin, unter alkalischen Bedingungen in organischen Lösungsmitteln hergestellt.

In der EP 0 005 129 A wird die Herstellung von 2-(2-Pyridinyl)methylthio-1 H-benzimidazolverbindungen durch Reaktion geeigneter Mercaptobenzimidazolverbindungen mit Chlormethylpyridinverbindungen beschrieben. Die Reaktion erfolgt in einem organischen Lösungsmittel, wie z.B. Ethanol, in Gegenwart von einer Base, wie z.B. Natriumhydroxid. Nach Beendigung der Reaktion wird das entstandene Kochsalz abgetrennt, das Lösungsmittel im Vakuum entfernt und das Monohydrochlorid der Verbindung mittels konzentrierter Salzsäure in Aceton zur Kristallisation gebracht und gereinigt. Bei diesem Verfahren erreicht man wenig zufriedenstellende Ausbeuten. Zudem muß das Hydrochlorid vor der Oxidationsreaktion wieder in die Base überführt werden.

In der EP 0 074 341 A wird die Herstellung von 2-(2-Pyridinyl)methylthio-1H-benzimidazolverbindungen durch Reaktion geeigneter Mercaptobenzimidazolverbindungen mit Chlormethylpyridinverbindungen in Gegenwart von Natriumhydroxid beschrieben. Als Lösungsmittel wird Methanol eingesetzt. Nach Beendigung der Reaktion und Zusatz von Wasser wird die 2-(2-Pyridinyl)methylthio-1 H-benzimidazolverbindung durch mehrmalige Extraktion mit Methylenchlorid und Umkristallisation aus Acetonitril gereinigt.
Bei diesem Verfahren werden umweltgefährdende Lösungsmittel eingesetzt. Zudem ist das Verfahren durch die mehrmaligen Extraktionsschritte zeitaufwendig.

In der EP 0 899 268 A2 wird u.a. die Herstellung von 2-[2-(4-Chlor-3,5-dimethylpyridyl) methylthio]-5-methoxy-1H-benzimidazol durch Reaktion geeigneter Ausgangsverbindungen in Tetrahydrofuran und in Gegenwart von Natriumhydroxidlösung beschrieben. Nach Beendigung der Reaktion und Zugabe von Wasser wird die genannte Verbindung durch mehrmalige Extraktion mit Methylenchlorid und Abdampfen des Lösungsmittels isoliert. Die Verbindung fällt als viskoses Öl an. Nachteilig an diesem Verfahren sind die Verwendung eines umweltgefährdenden Lösungsmittels und die zeitaufwendige Isolierung durch mehrfache Extraktion. Zudem ist das Produkt als viskoses Öl verfahrenstechnisch schlechter zu handhaben als eine kristalline Verbindung.

Aufgabe der vorliegenden Erfindung war es daher, die gegebenenfalls substiuierten 2-(2-Pyridinyl)methylthio-1 H-benzimidazole in einer Form zur Verfügung zu stellen, die stabil und lagerfähig ist und die durch einfache verfahrenstechnische Schritte in hohen Ausbeuten und mit großer Reinheit erhalten wird, wobei umwelt- und gesundheitsverträglichere Lösungsmittel eingesetzt werden können.

Die Aufgabe wird durch Bereitstellung von Kristallen aus gegebenenfalls substiuierten 2-(2-Pyridinyl)methylthio-1 H-benzimidazol-Hydraten der Strukturformel I gelöst, 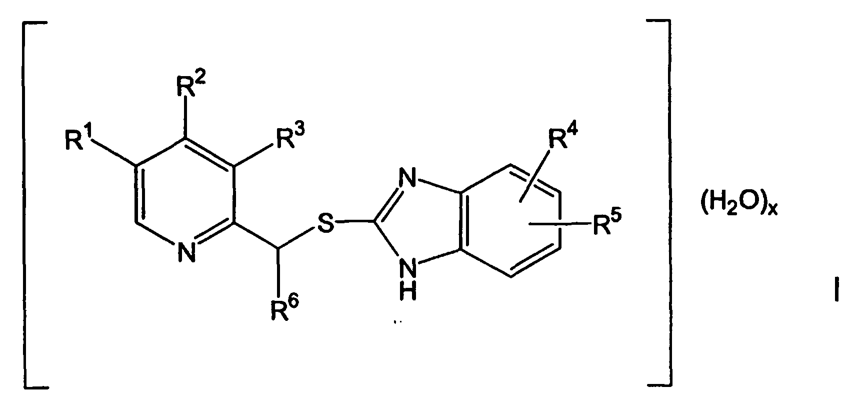 worin
- R¹, R² und R³,: gleich oder verschieden, für Wasserstoff, C1-C8-Alkyl, C3-C8-Cycloalkyl, C2-C8-Fluoralkyl oder C1-C8-Alkoxy,
- R⁴ und R⁵,: gleich oder verschieden, für Wasserstoff, C1-C8-Alkyl, C3-C8-Cycloalkyl, CH₂-C3-C8- Cycloalkyl, C1-C8-Alkoxycarbonyl, C1-C8-Alkoxy, C1-C8-Fluoralkoxy, CF₃, C2-C8-Fluoralkyl oder -C(O)O-C1-C8-Alkyl und
- R⁶,: gleich oder verschieden, für Wasserstoff oder C1-C2-Alkyl und
- x: für 0.5 - 2 steht.

Vorzugsweise haben R¹ - R⁶ folgende Bedeutung
- R¹, R² und R³,: gleich oder verschieden, für Wasserstoff, C1-C3-Alkyl oder C1-C3-Alkoxy,
- R⁴ und R⁵,: gleich oder verschieden, für Wasserstoff, C1-C3-Alkoxy, C1-C3-Fluoralkoxy und
- R⁶,: gleich oder verschieden, für Wasserstoff steht.

Besonders bevorzugt sind Verbindungen,
worin R¹ für eine Methylgruppe, R² für eine Methoxygruppe, R³ für eine Methylgruppe, R⁴ für Wasserstoff, R⁵ für Methoxygruppe in 5 Position und R⁶ für Wasserstoff steht oder
worin R¹ für Wasserstoff, R² und R³ jeweils für eine Methoxygruppe, R⁴ für Wasserstoff, R⁵ für Difluormethoxygruppe in 5 Position und R⁶ für Wasserstoff steht.

Ein weiterer Gegenstand der vorliegenden Erfindung ist ein Verfahren zur Isolierung einer Verbindung gemäß Formel I aus einem Reaktionsmedium, in dem die Verbindung als freie Base vorliegt, mit hohen Ausbeuten, wobei ein im Reaktionsmedium vorhandenes mit Wasser mischbares, organisches Lösungsmittel höchstens teilweise entfernt und dem Reaktionsmedium bei einer Temperatur unterhalb 40°C, vorzugsweise 20-25°C, Wasser in Mengen von mindestens 55 Gew.-%, vorzugsweise mindestens 70 Gew.%, besonders bevorzugt bis 75 Gew.-% bezogen auf das Reaktionsmedium, zugesetzt wird, und die dabei gebildeten Hydrate als Kristalle abgetrennt und gegebenenfalls in üblicher Weise gereinigt und getrocknet werden. Bei der Entfernung des organischen Lösungsmittels ist darauf zu achten, daß die Löslichkeitsgrenze der Verbindung der Formel I nicht unterschritten wird.

Vorzugsweise sind die Verbindungen der Formel I aus einem Reaktionsmedium abzutrennen, daß man durch Umsetzung einer Thiol-Verbindung der Formel II, 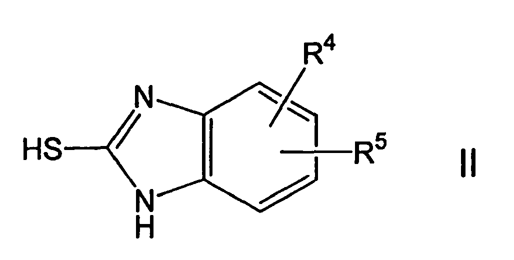 worin R⁴ und R⁵ die oben angegebene Bedeutung haben, mit einer reaktiven Pyridin- Verbindung der Formel III, 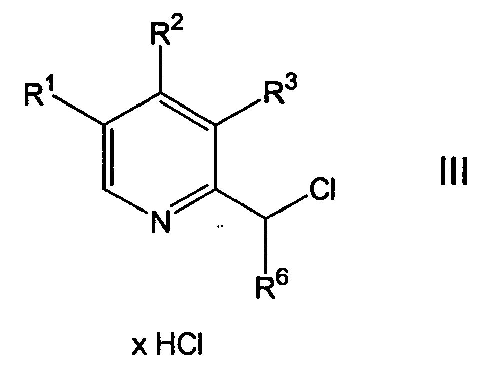 worin R¹, R², R³ und R⁶ die oben angegebene Bedeutung haben, in einem mit Wasser mischbaren, organischen Lösungsmittel in Anwesenheit einer Base erhält. Geeignete Basen bei dieser Umsetzung sind vorzugsweise Natrium- und/oder Kaliumhydroxid. Vorzugsweise erfolgt diese Umsetzung bei mehrstündigem Kochen unter Rückfluß. Die Reaktion kann kontinuierlich oder diskontinuierlich geführt werden.

Aus dem Reaktionsmedium, das die Verbindung als freie Base enthält, kann die Verbindung nach Formel I durch höchstens teilweises Entfernen des mit Wasser mischbaren, organischen Lösungsmittels und durch Zusatz von Wasser in Mengen von mindestens 55 Gew.-%, vorzugsweise mindestens 70 Gew.-%, besonders bevorzugt bis 75 Gew.-% bezogen auf das Reaktionsmedium bei einer Temperatur unterhalb von 40°C, vorzugsweise 20-25°C, gewonnen werden. Bei der Entfernung des organischen Lösungsmittels ist darauf zu achten, daß die Löslichkeitsgrenze der Verbindung der Formel 1 nicht unterschritten wird.

Das durch die Neutralisation der zugesetzten Base entstandene Natrium- und/oder Kaliumchlorid kann zuvor durch geeignete Maßnahmen, z.B. Filtration, abgetrennt oder durch den Zusatz des Wassers bei der Hydratbildung gelöst werden. Gegebenenfalls können die Hydrate der 2-(2-Pyridinyl)methylthio-1 H-benzimidazole weiter aufgereingt und getrocknet werden.

Thiol-Verbindungen der Formel II können käuflich erworben werden. Zur Beschreibung ihrer Synthese wird auch auf EP 0 254 588, EP 0 005 129 und EP 0 074 341 verwiesen, wobei die entsprechende Beschreibung hiermit als Teil der vorliegenden Offenbarung eingeführt wird. Zur Beschreibung der Synthese der reaktiven Pyridin-Verbindungen der Formel III wird auf WO 98/50361 und WO 97/29103 verwiesen, wobei die entsprechende Beschreibung hiermit als Teil der vorliegenden Offenbarung eingeführt wird.

Ein weiterer Gegenstand der vorliegenden Erfindung ist ein Verfahren zur Herstellung einer Verbindung gemäß Formel I, bei dem man die nicht-hydratisierte Verbindung nach Formel I in einem mit Wasser mischbaren, organischen Lösungsmittel oder Lösungsmittelgemisch löst und durch Zusatz von Wasser in Mengen von mindestens 55 Gew.-%, vorzugsweise mindestens 70 Gew.-%, besonders bevorzugt bis 75 Gew.% bezogen auf das Reaktionsmedium bei einer Temperatur unterhalb von 40°C, vorzugsweise 20-25°C, zur Kristallisation bringt und die dabei gebildeten Kristalle der Verbindung gemäß Formel abtrennt, gegebenenfalls reinigt und trocknet.

Als mit Wasser mischbare, organische Lösungsmittel werden in den vorstehend genannten Reaktionen bevorzugt leicht flüchtige Lösungsmittel, wie aliphatische Alkohole, aprotische Lösungsmittel oder Ketone, besonders bevorzugt Methanol, Ethanol, Propanol, Butanol, Dimethylformamid, Dimethylsulfoxid, Tetrahydrofuran oder Aceton, oder Mischungen aus wenigstens zwei dieser Lösungsmittel eingesetzt.

Ein weiterer Gegenstand der vorliegenden Erfindung ist auch ein Verfahren zur Reinigung von Kristallen einer Verbindung gemäß Formel 1, gemäß dem das zu reinigende Hydrat mit Wasser und/oder einem Lösungsmittel-Wassergemisch, vorzugsweise einem Alkohol-Wassergemisch und/oder einem Keton-Wassergemisch, wenigstens einmal gewaschen und anschließend im Vakuum unterhalb des Schmelzpunktes der Hydrate getrocknet wird.

Die erfindungsgemäßen Kristalle aus den gegebenfalls substituierten 2-(2-Pyridinyl)methylthio-1 H-benzimidazol-Hydraten der Formel I sind stabil und lagerfähig. Sie sind verfahrenstechnisch einfach herzustellen, zu isolieren und reinigen und können direkt für die Oxidation zu den entsprechenden Sulfinylverbindungen, die als Ulkustherapeutikum Verwendung finden, eingesetzt werden. Mit Hilfe der erfindungsgemäßen Verfahren gelingt es, Kristalle aus gegebenenfalls substituierten 2-(2-Pyridinyl)methylthio-1 H-benzimidazol-Hydraten der Formel I in hohen Ausbeuten und mit großer Reinheit herzustellen, wobei umwelt- und gesundheitsverträglichere Lösungsmittel eingesetzt werden können.

Im folgenden wird die Erfindung durch Beispiele erläutert, ohne sie darauf zu beschränken.

### Beispiele

### Beispiel 1

Zu einer Lösung von 0.11 mol Natriumhydroxid in 90 ml Ethanol wurden 0.05 mol 2-Mercapto-5-methoxybenzimidazol gegeben. Der Lösung wurden 0.05 mol 2-Chlormethyl-3,5-dimethyl-4-methoxypyridin-Hydrochlorid zugesetzt und die Reaktionsmischung 14 Stunden unter Rückfluß gekocht. Anschließend wurden bei Raumtemperatur (25°C) 270 ml Wasser hinzugefügt, wobei es zur Kristallisation der Hydrate von 5-Methoxy-2-[3,5-dimethyl-4-methoxypyridinyl)methylthio]-1H-benzimidazol kam. Das weißliche Kristallisat wurde abgetrennt, mit Wasser gewaschen und im Vakuum getrocknet. Ausbeute: 95 % bezogen auf die theoretische Ausbeute (15.6 g).

Die Reinheit der Verbindung wurde durch HPLC bestimmt und war 99,7 %.

### Beispiel 2

Zu einer Lösung von 0.22 mol Natriumhydroxid in 250 ml Methanol wurden 0.1 mol 2-Mercapto-5-methoxybenzimidazol gegeben. Der Lösung wurden 0.1 mol 2-Chlormethyl-3,5-dimethyl-4-methoxypyridin-Hydrochlorid zugesetzt und die Reaktionsmischung 16 Stunden unter Rückfluß gekocht. Anschließend wurden im Vakuum 80 ml Lösungsmittel entfernt und danach bei Raumtemperatur (25°C) 400 ml Wasser hinzugefügt, wobei es zur Kristallisation der Hydrate von 5-Methoxy-2-[3,5-dimethyl-4-methoxypyridinyl)methylthio]-1H-benzimidazol kam. Das weißliche Kristallisat wurde abgetrennt, mit einem Methanol/Wasser gewaschen und im Vakuum getrocknet.
Ausbeute: 92.5 % bezogen auf die theoretische Ausbeute (30.3 g).
Die Reinheit der Verbindung wurde durch HPLC bestimmt und war 99,5 %.

### Beispiel 3

0.1 mol Pyrmetazol-Hydrochlorid (die Verbindung wurde gemäß den Angaben von Beispiel 31 der EP 0 005 129 hergestellt) wurden in 60 ml Wasser gelöst, anschließend wurden 60 ml Ethanol zugegeben, der pH Wert der Lösung wurde mit einer 5 N Natriumhydroxid Lösung auf größer pH 7 eingestellt und bei Raumtemperatur (25°C) wurden weitere 120 ml Wasser hinzugefügt, wobei es zur Kristallisation der Hydrate des Pyrmetazols kam. Das weißliche Kristallisat wurde abgetrennt, mit Wasser gewaschen und im Vakuum getrocknet. Ausbeute: 90.2 % bezogen auf die theoretische Ausbeute (29.5 g).
Die Reinheit der Verbindung wurde durch HPLC bestimmt und war 99,9 %.

### Beispiel 4

Zunächst wurde gemäß Beispiel 26 der EP 0 899 268 Pyrmetazol hergestellt, das als Lösung in Methylenchlorid vorlag. Eine solche Lösung, die 0.1 Mol Pyrmetazol enthielt, wurde im Vakuum vom Methylenchlorid befreit.

Anschließend wurde das zurückgebliebene Öl in 210 ml Ethanol gelöst und durch Zugabe von 630 ml Wasser als Hydrat von Pyrmetazol zur Kristallisation gebracht. Das weißliche Kristallisat wurde abgetrennt, mit Wasser gewaschen und im Vakuum getrocknet.
Ausbeute: 96 % bezogen auf die theoretische Ausbeute (31.5 g).
Die Reinheit der Verbindung wurde durch HPLC bestimmt und war 99,8 %.

## Patentansprüche

1. Kristalle aus gegebenenfalls substituierten 2-(2-Pyridinyl)methylthio-1 H-benzimidazol-Hydraten der folgenden Strukturformel I 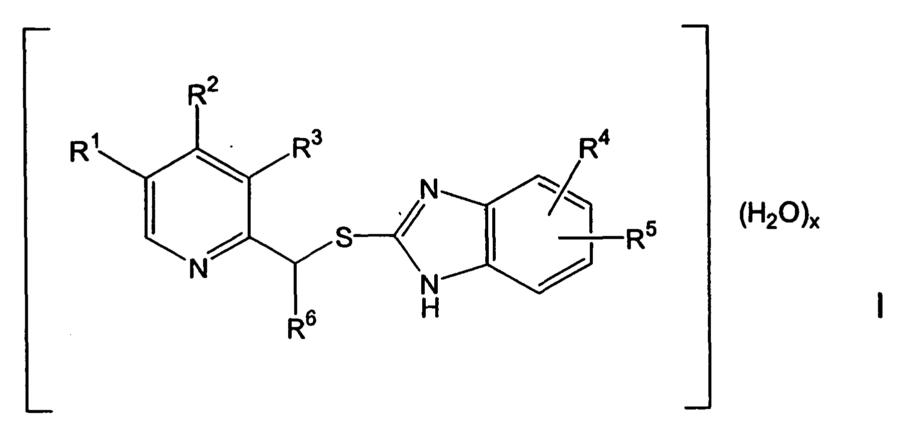
worin R¹, R² und R³, gleich oder verschieden, für Wasserstoff, einen C1-C8-Alkyl-, C3-C8-Cycloalkyl-, C2-C8-Fluoralkyl- oder C1-C8-Alkoxy-Rest,
R⁴ und R⁵, gleich oder verschieden, für Wasserstoff, einen C1-C8-Alkyl-, C3-C8-Cycloalkyl-, CH₂-C3-C8-Cyloalkyl-, C1-C8-Alkoxycarbonyl-, C1-C8-Alkoxy-, C1-C8-Fluoralkoxy-, CF₃-, C2-C8-Fluoralkyl- oder C(O)O-C1-C8-AlkylRest und
R⁶ für Wasserstoff oder einen C1-C2-Alkyl-Rest steht und
x = 0.5 - 2 bedeutet.

2. Kristalle nach Anspruch 1,
worin R¹, R² und R³, gleich oder verschieden, für Wasserstoff, einen C1-C3-Alkyl- oder C1-C3-Alkoxy-Rest,
R⁴ und R⁵, gleich oder verschieden, für Wasserstoff, einen C1-C3-Alkoxy-, C1-C3-Fluoralkoxy-Rest und
R⁶ für Wasserstoff steht und
x = 0.5 - 2 bedeutet.

3. Kristalle nach Anspruch 1 oder 2,
worin R¹ für eine Methylgruppe, R² für eine Methoxygruppe, R³ für eine Methylgruppe, R⁴ für Wasserstoff, R⁵ für Methoxygruppe in 5 Position und R⁶ für Wasserstoff steht und
x = 0.5 - 2 bedeutet.

4. Kristalle nach Anspruch 1 oder 2,
worin R¹ für eine Wasserstoff, R² und R³ jeweils für eine Methoxygruppe, R⁴ für Wasserstoff, R⁵ für Difluormethoxygruppe in 5 Position und R⁶ für Wasserstoff steht und
x = 0.5 - 2 bedeutet.

5. Verfahren zur Isolierung einer Verbindung gemäß einem der Ansprüche 1-4 aus einem Reaktionsmedium, in dem die freie Base vorliegt, **dadurch gekennzeichnet, daß** ein im Reaktionsmedium vorhandenes, mit Wasser mischbares, organisches Lösungsmittel höchstens teilweise entfernt, dem Reaktionsmedium bei einer Temperatur unterhalb von 40°C Wasser in Mengen von mindestens 55 Gew.-% bezogen auf das Reaktionsmedium zugesetzt wird und die gebildeten Hydrate als Kristalle abgetrennt und ggf. in üblicher Weise gereinigt werden.

6. Verfahren gemäß Anspruch 5, **dadurch gekennzeichnet, daß** Wasser in Mengen von mindestens 70 Gew.-% bezogen auf das Reaktionsmedium zugesetzt wird.

7. Verfahren gemäß Anspruch 5, **dadurch gekennzeichnet, daß** Wasser in Mengen bis zu 75 Gew.-% bezogen auf das Reaktionsmedium zugesetzt wird.

8. Verfahren gemäß einem der Ansprüche 5 - 7, **dadurch gekennzeichnet, daß** die Wasserzugabe bei einer Temperatur von 20-25°C erfolgt.

9. Verfahren nach einem der Ansprüche 5-8, **dadurch gekennzeichnet, daß** in dem Reaktionsmedium eine nicht-hydratisierte Verbindung nach Formel I durch Umsetzung einer Thiol-Verbindung nach Formel 11 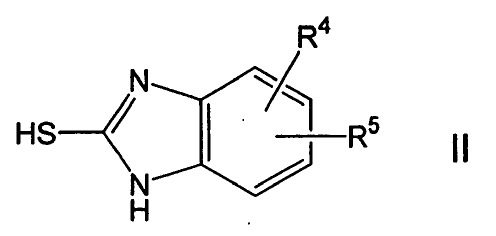 mit einer reaktiven Pyridin-Verbindung nach Formel III 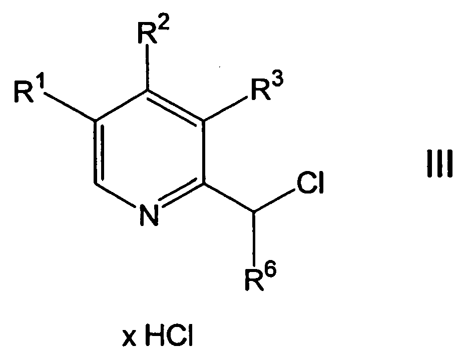 in Anwesenheit mindestens einer Base erhalten wurde, wobei die Reste R¹ - R⁶ die in einem der Ansprüche 1 - 4 angegebene Bedeutung haben.

10. Verfahren nach Anspruch 9, **dadurch gekennzeichnet, daß** als Base Natrium- und/oder Kaliumhydroxid eingesetzt wurde.

11. Verfahren nach einem der Ansprüche 5 - 8, **dadurch gekennzeichnet, daß** die nicht-hydratisierte Verbindung nach Formel I zunächst in einem mit Wasser mischbaren, organischen Lösungsmittel gelöst wurde.

12. Verfahren nach einem der Ansprüche 5 -11, **dadurch gekennzeichnet, daß** das mit Wasser mischbare, organische Lösungsmittel ein aliphatischer Alkohol, vorzugsweise Methanol, Ethanol, Propanol oder Butanol, oder ein aprotisches Lösungsmittel, vorzugsweise Dimethylformamid, Dimethylsulfoxid, Tetrahydrofuran, oder ein Keton, vorzugsweise Aceton, oder eine Mischung von wenigstens zwei dieser Lösungsmittel ist.

13. Verfahren nach einem der Ansprüche 5 -12, **dadurch gekennzeichnet, daß** die Kristalle mit Wasser und/oder einem Lösungsmittel-Wassergemisch, vorzugsweise einem Alkohol-Wassergemisch und/oder einem Keton-Wassergemisch zur Reinigung gewaschen werden.

## Claims

1. Crystals of optionally substituted 2-(2-pyridinyl)-methylthio-1H-benzimidazole hydrates of the following structural formula I 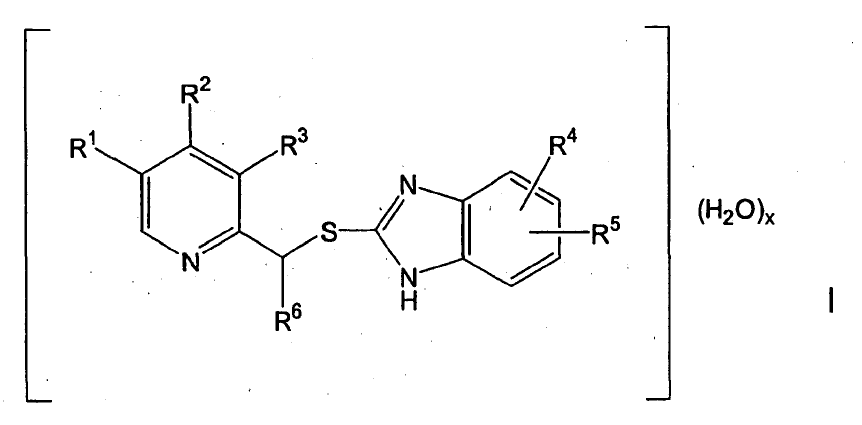 in which R¹, R² and R³, identical or different, denote hydrogen, a C1-C8 alkyl, C3-C8 cycloalkyl, C2-C8 fluoroalkyl or C1-C8 alkoxy residue,
R⁹ and R⁵, identical or different, denote
hydrogen, a C1-C8 alkyl, C3-C8 cycloalkyl, CH₂-C3-C8 cycloalkyl, C1-C8 alkoxycarbonyl, C1-C8 alkoxy, C1-C8 fluoroalkoxy, CF₃-, C2-C8 fluoroalkyl or C(O)O-C1-C8 alkyl residue and
R⁶ denotes
hydrogen or a C1-C2 alkyl residue and
x means 0.5-2.

2. Crystals according to claim 1,
in which R¹, R² and R³, identical or different, denote hydrogen, a C1-C3 alkyl or C1-C3 alkoxy residue,
R⁹ and R⁵, identical or different, denote hydrogen, a C1-C3 alkoxy, C1-C3 fluoroalkoxy residue and
R⁶ denotes
hydrogen and
x means 0.5-2.

3. Crystals according to claim 1 or 2,
in which R¹ denotes a methyl group, R² a methoxy group, R³ a methyl group, R⁴ hydrogen, R⁵ a methoxy group in position 5 and R⁶ hydrogen and
x means 0.5-2.

4. Crystals according to claim 1 or 2,
in which R¹ denotes hydrogen, R² and R³ in each case denote a methoxy,group, R⁴ denotes hydrogen, R⁵ a difluoromethoxy group in position 5 and R⁶ hydrogen and x means 0.5-2.

5. A process for the isolation of a compound according to one of claims 1-4 from a reaction medium containing the free base, **characterised in that** a water-miscible, organic solvent present in the reaction medium is at most partially removed, water is added to the reaction medium at a temperature of below 40°C in quantities of at least 55 wt.%, relative to the reaction medium, and the hydrates formed are separated as crystals and optionally purified in conventional manner.

6. A process according to claim 5, **characterised in that** water is added in quantities of at least 70 wt.% relative to the reaction medium.

7. A process according to claim 5, **characterised in that** water is added in quantities of up to 75 wt.% relative to the reaction medium.

8. A process according to one of claims 5-7, **characterised in that** the water is added at a temperature of 20-25°C.

9. A process according to one of claims 5-8, **characterised in that** an unhydrated compound of the formula I was obtained in the reaction medium by reacting a thiol compound of the formula II 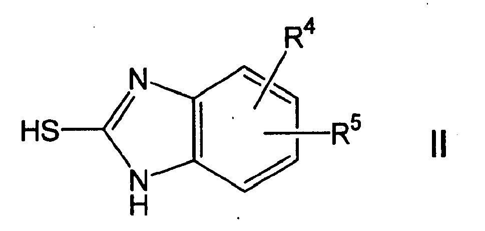 with a reactive pyridine compound of the formula III 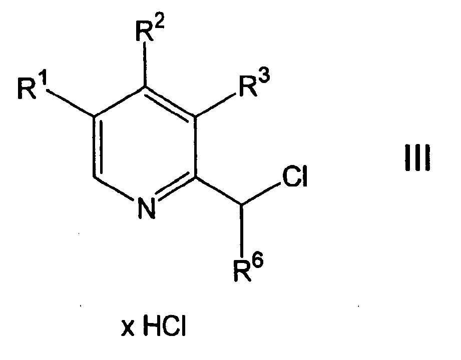 in the presence of at least one base, wherein the residues R¹-R⁶ have the meaning stated in one of claims 1-4.

10. A process according to claim 9, **characterised in that** sodium hydroxide and/or potassium hydroxide was used as the base.

11. A process according to one of claims 5-8, **characterised in that** the unhydrated compound of the formula I was initially dissolved in a water-miscible, organic solvent.

12. A process according to one of claims 5-11, **characterised in that** the water-miscible, organic solvent is an aliphatic alcohol, preferably methanol, ethanol, propanol or butanol, or an aprotic solvent, preferably dimethylformamide, dimethyl sulfoxide, tetrahydrofuran, or a ketone, preferably acetone, or a mixture of at least two these solvents.

13. A process according to one of claims 5-12, **characterised in that** the crystals are purified by washing with water and/or a solvent/water mixture, preferably an alcohol/water mixture and/or a ketone/water mixture.

## Revendications

1. Cristaux d'hydrates de 2-(2-pyridinyl)méthylthio-1H-benzimidazole le cas échéant substitués présentant la formule de structure I suivante 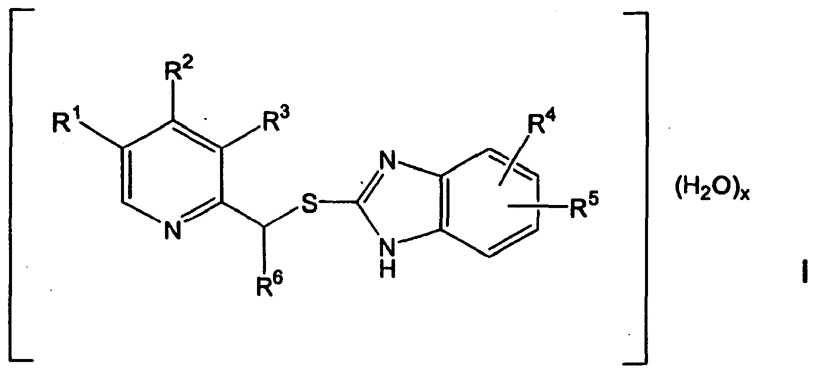 dans laquelle
R¹, R² et R³ sont identiques ou différents et représentent
hydrogène, un radical C1-C8-alkyle, C3-C8-cycloalkyle, C2-C8-fluoroalkyle ou C1-C8-alcoxy,
R⁴ et R⁵ sont identiques ou différents et représentent hydrogène, un radical C1-C8-alkyle, C3-C8-cycloalkyle, CH₂-C3-C8-cycloalkyle, C1-C8-alcoxycarbonyle, C1-C8-alcoxy, C1-C8-fluoroalcoxy, CF₃, C2-C8-fluoroalkyle ou C(O)O-Cl-C8-alkyle et
R⁶ représente
hydrogène ou un radical C1-C2-alkyle et
x = 0,5 - 2.

2. Cristaux selon la revendication 1,
dans laquelle R¹, R² et R³ sont identiques ou différents et représentent
hydrogène, un radical C1-C3-alkyle ou C1-C3- alcoxy,
R⁹ et R⁵, sont identiques ou différents et représentent hydrogène, un radical C1-C3-alcoxy, C1-C3-fluoroalcoxy et
R⁶ représente
hydrogène et
x = 0,5 - 2.

3. Cristaux selon la revendication 1 ou 2,
où R¹ représente un groupe méthyle, R² représente un groupe méthoxy, R³ représente un groupe méthyle, R⁴ représente hydrogène, R⁵ représente un groupe méthoxy en 5ème position et R⁶ représente hydrogène et
x = 0,5 - 2.

4. Cristaux selon la revendication 1 ou 2,
où R¹ représente hydrogène, R² et R³ représentent à chaque fois un groupe méthoxy, R⁴ représente hydrogène, R⁵ représente un groupe difluorométhoxy en 5ème position et R⁶ représente hydrogène et
x = 0,5 - 2.

5. Procédé pour isoler un composé selon l'une quelconque des revendications 1-4 d'un mélange réactionnel, dans lequel se trouve la base libre, **caractérisé en ce qu'**on élimine au plus partiellement un solvant organique présent dans le mélange réactionnel, miscible avec l'eau, on ajoute au mélange réactionnel, à une température inférieure à 40°C, de l'eau en des quantités d'au moins 55% en poids par rapport au mélange réactionnel et on sépare les hydrates formés sous forme de cristaux et on les purifie le cas échéant de manière usuelle.

6. Procédé selon la revendication 5, **caractérisé en ce qu'**on ajoute de l'eau en des quantités d'au moins 70% en poids par rapport au mélange réactionnel.

7. Procédé selon la revendication 5, **caractérisé en ce qu'**on ajoute de l'eau en des quantités jusqu'à 75% en poids par rapport au mélange réactionnel.

8. Procédé selon l'une quelconque des revendications 5-7, **caractérisé en ce** l'addition d'eau est réalisée à une température de 20-25°C.

9. Procédé selon l'une quelconque des revendications 5-8, **caractérisé en ce qu'**on obtient dans le mélange réactionnel un composé non hydraté selon la formule I par transformation d'un composé de type thiol selon la formule II 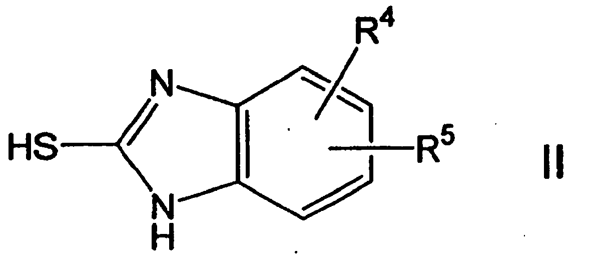 avec un composé réactif de type pyridine de formule III 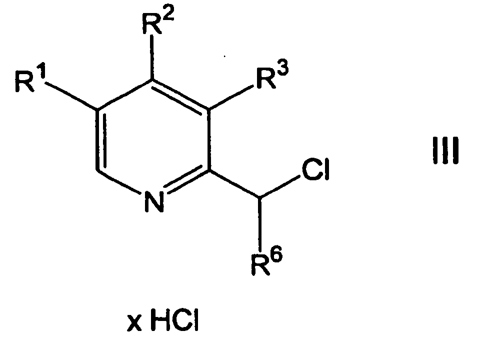 en présence d'au moins une base, les radicaux R¹ - R⁶ ayant la signification indiquée dans l'une quelconque des revendications 1-4.

10. Procédé selon la revendication 9, **caractérisé en ce qu'**on utilise comme base de l'hydroxyde de sodium et/ou de potassium.

11. Procédé selon l'une quelconque des revendications 5-8, **caractérisé en ce que** le composé non hydraté de formule I a d'abord été dissous dans un solvant organique miscible avec l'eau.

12. Procédé selon l'une quelconque des revendications 5-11, **caractérisé en ce que** le solvant organique miscible avec l'eau est un alcool aliphatique, de préférence le méthanol, l'éthanol, le propanol ou le butanol, ou un solvant aprotique, de préférence le diméthylformamide, le diméthylsulfoxyde, le tétrahydrofuranne, ou une cétone, de préférence l'acétone, ou un mélange d'au moins deux de ces solvants.

13. Procédé selon l'une quelconque des revendications 5-12, **caractérisé en ce que** les cristaux sont lavés avec de l'eau et/ou un mélange solvant/eau, de préférence un mélange alcool-eau et/ou un mélange cétone-eau en vue de leur purification.
